# EUROPÄISCHE PATENTSCHRIFT

(11) **EP 1 319 390 B1**
(45) Veröffentlichungstag und Bekanntmachung des Hinweises auf die Patenterteilung: **23.06.2010**
(21) Anmeldenummer: 02026711.8
(22) Anmeldetag: 30.11.2002
(51) Int. Cl.: A61K 8/04, A61K 8/41, A61K 8/81, A61Q 5/06

(54) **Haarfestiger mit kationischer Substanz**
Composition for fixing the hair with a cationic compound
Composition capillaire fixante avec une substance cationique

(30) Priorität: 12.12.2001 DE 10160991
(43) Veröffentlichungstag der Anmeldung: 18.06.2003
(73) Patentinhaber: Beiersdorf AG, 20245 Hamburg (DE)
(72) Erfinder: Primmel, Bettina, 20146 Hamburg (DE); Liebelt, Kerstin, 22529 Hamburg (DE)

(56) Entgegenhaltungen:
- EP-A2- 1 072 250
- WO-A1-00/68282
- WO-A1-96/19966
- DE-A1- 19 937 386
- DE-C1- 19 957 947
- US-A1- 2001 026 791

## Beschreibung

Die vorliegende Erfindung betrifft Haarfestiger enthaltend mindestens ein Terpolymer aus Vinylpyrrolidon, Dimethylaminopropylmethacrylamid und quaternisiertem Alkyldimethylaminopropylmethacrylamid in einer Konzentration von 0,1 bis 10 Gewichts-% und mindestens einer kationischen Substanz nach Anspruch 1 in einer Konzentration von 0,01 bis 20 Gewichts-%, jeweils bezogen auf das Gesamtgewicht der Zubereitung und deren Verwendung.

Der ganze menschliche Körper mit Ausnahme der Lippen, der Handinnenflächen und der Fußsohlen ist behaart, zum Großteil allerdings mit kaum sichtbaren Wollhärchen. Wegen der vielen Nervenenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt es eine soziale Funktion, da es über sein Erscheinungsbild erheblich zum Selbstwertgefühl des Individuums beiträgt.

Das Haar besteht aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft seinerseits ist aus drei Schichten aufgebaut: einem zentralen Teil - dem sogenannten Haarmark (Medulla), welches allerdings beim Menschen zurückgebildet ist und oft gänzlich fehlt - ferner dem Mark (Cortex) und der äußeren, bis zu zehn Lagen starken Schuppenschicht (Cuticula), die das ganze Haar umhüllt.

Natürliches Haar hängt meist schlaff und ohne Volumen vom Kopf herab. Ein Ziel der Haarpflege ist es daher, dem Haar Fülle und Frisur zu geben. Zur Gestaltung (engl. Styling) vielseitiger Frisuren verwendet man Haarfestiger, meist in Form von Schaumfestigern oder Haarsprays. Beide haarkosmetischen Mittel sind in Ihrer Zusammensetzung ähnlich unterscheiden sich aber in ihrer Aufgabe und Anwendung.

Schaumfestiger (auch Haarschaumfestiger genannt) werden in der Regel zur Frisurgestaltung im feuchten Haar verteilt, Haarsprays zur Fixierung auf die (trockene) fertig gestylte Frisur gesprüht. Neben den wegen ihrer leichten und angenehmen Anwendbarkeit zunehmend beliebten Schaumhaarfestigem und flüssigen Haarfestigern werden auch Haarfestigergele angeboten.

Die beiden Grundbausteine von Haarfestigern sind die Filmbildner und das Lösungsmittel. Als Filmbildner werden meist Mischpolymerisate aus Vinylpyrrolidon und Vinylacetat oder neutralisierter Crotonsäure eingesetzt. Filmbildner bleiben nach dem Verdunsten des Lösungsmittels (meist Wasser und/oder Ethanol) als Film auf dem Haar und fixieren dessen Form. Haarschaumfestiger enthalten zusätzlich Treibmittel wie Propan, n-Butan und/oder Isobutan.

Je nach gewünschter Festigkeit der Frisur werden unterschiedlich stark festigende Filmbildner in unterschiedlichen Mengenanteilen eingesetzt. Man unterscheidet daher Haarfestiger mit schwacher, normaler und starker Festigung sowie Festiger für normales, trockenes und fettiges Haar [W. Umbach (Hrsg.): Kosmetik, Entwicklung, Herstellung und Anwendung kosmetischer Mittel, 2. Aufl., Thieme Verlag, Stuttgart, 1995].

Herkömmliche Haarfestiger, die nur kationische Polymere als Filmbildner einsetzen, haben den Nachteil, dass sie den Anforderungen bezüglich ihrer sensorischen Eigenschaften (insbesondere geringe Klebrigkeit, der Schaum fühlt sich "glitschig"an) und ihrer Schaumcharakteristik (insbesondere Stabilität, Verteilbarkeit im Haar, etc. bei aufzuschäumenden Produkten) nur unzureichend gerecht werden. Auch der "Griff" des nassen und trockenen Haares läßt nach einer Behandlung mit herkömmlichen Haarfestigern zu wünschen übrig.

Es war daher die Aufgabe der vorliegenden Erfindung, den Nachteilen des Standes der Technik abzuhelfen und Haarfestiger zu entwickeln, deren sensorische Eigenschaften und Schaumcharakteristik denen herkömmlicher Produkte überlegen ist.

Überraschend wird die Aufgabe gelöst durch Haarfestiger enthaltend
a) mindestens ein Terpolymer aus Vinylpyrrolidon, Alkyldimethylaminopropylmethacrylamid und quaternisiertem Alkyldimethylaminopropylmethacrylamid in einer Konzentration von 0,1 bis 10 Gewichts-%
b) mindestens eine kationische Substanz in einer Konzentration von 0,01 bis 20 Gewichts-%, jeweils bezogen auf das Gesamtgewicht der Zubereitung, dadurch gekennzeichnet, dass die kationische Substanz ein kationisches, betainisches oder amphoteres Tensid, eine Silikonverbindung mit kationischen Gruppen, Cetyltrimethylammoniumchlorid und/oder Cetyltrimethylammoniumphosphat, ein kationisch derivatisiertes Protein oder ein kationisch derivatisiertes hydrolysiertes Protein ist sowie **dadurch gekennzeichnet, dass** die Zubereitung mit einem Treibgas aufgeschäumt wird, wobei als Treibgas Propan, Isobutan und/oder n-Butan eingesetzt wird.

Unter kationischen Substanzen versteht man erfindungsgemäß Verbindungen, die mindestens eine kationische Funktion, meist einen quaternisiertes Stickstoffatom, enthalten, wie kationische, betainische oder amphotere Tenside, kationische Pflege-Polymere, Silikonverbindungen mit kationischen Gruppen, kationisch derivatisierte Proteine oder kationisch derivatisierte hydrolysierte Proteine

Zwar beschreibt die WO 0068282 Terpolymere aus Vinylpyrrolidon, Dimethylaminopropylmethacrylamid und quaternisierten Alkyldimethylaminopropylmethacrylamid in Haarstylingprodukten, die sich durch gute Filmeigenschaften wie geringe Klebrigkeit und eine gute Feuchtigkeitsresistenz auszeichnen. Doch ließ bei den hier offenbarten Produkten die für die Verbraucherakzeptanz so wichtige Schaumqualität zu wünschen übrig.

Auch andere kationische Polymere [z.B. Polyquaternium-4 (Hydroxyethylcellulosedimethyldiallylammoniumchlorid-Copolymer), Polyquaternium-11 (quaternisiertes Vinylpyrrolidonalkylaminomethacrylat) oder Polyquaternium-16 (Methylvinylimidazoliumvinylpyrrolidon)], sind als filmbildene Bestandteile von Haarfestigungsprodukten an sich bekannt, da sie eine hohe Pflegeleistung besitzen. Ihre Festigungsleistung ist jedoch unzureichend, weshalb sie in Abmischungen mit anderen, meist anionischen, amphoteren oder nichtionischen Filmbildnern, eingesetzt werden. Auch die Schaumqualität leidet unter Einsatz größerer Mengen an kationischen Polymeren.

Nicht zuletzt waren dem Fachmann die Schriften DE 19937386, WO 96/19966, WO 00/68282, US 2001/026791, EP 1072250, DE 19957947 und Soap & Cosmetics, October 2001, Seite 40 bekannt, doch konnten diese Schriften nicht den Weg zur vorliegenden Erfindung weisen.

WO-A-02/058649 offenbart einen Haarfestiger enthaltend ein fixierendes Polymer und ein kationisches Poly(vinyllactam). Das fixierende Polymer ist möglicherweise ein amphoteres oder zwitterionisches Polymer.

Soap & Cosmetics, October 2001, page 40 offenbart ein "Styling Mousse", das 5.00 Gew.% Gafquat 755N (kationische Polyquaternium-11) und 5.00 Gew.% kationische Styleze W-20 (Polyquaternium-55: Terpolymer aus Vonylpyrrolidon, Dimethylaminopropylmethacrylamid und quaternisiertem Alkyldimethylaminopropylmethacrylamid) enthält.

DE 199 37 386 beschreibt ein Haarfestiger mit einem Gehalt an (A) mindestens einem Terpolymeren aus Vinylpyrrolidon, Vinylcaprolactam und einem basischen Acrylamidmonomer und (B) mindestens einem haarpflegenden Stoff mit mindestens einer kationaktiven Gruppe (Seite 2, Zeilen 26-43). Die Beispiele zeigen Schaumfestiger sowie auch eine Sprühlotion.

WO 96/19966 offenbart Vinyllactam-basierte Terpolymere zur Verwendung in Haarpflegemittel.

US 2001/026791 offenbart Haarpflegemittel enthaltend die Terpolymere aus DE 199 37 386, gelbildende Zutaten und weitere filmbildende Polymere.

EP1 072 250 offenbart ein wasserhaltiges Aerosol-Haarspray enthaltend haarfestigende Terpolymere wie die aus DE 199 37 386 bekannten Terpolymere.

DE 199 57 947 offenbart Haarpflegemittel enthaltend ein Polymerkombination aus (A) mindestens einem Terpolymeren aus Vinylpyrrolidon, Vinylcaprolactam und einem basischen Acrylamidmonomer und (B) mindestens einem Polymeren mit anionischen oder anionisierbaren Gruppen.

Es war daher für den Fachmann nicht vorhersehbar, dass die Kombination der beiden Bestandteile der erfindungsgemäßen Zubereitung in synergistischer Weise zu Haarfestigern führt, deren Schaum so cremig, samtig und feinporig ist. Der Schaum hat eine stabile Konsistenz, läßt sich einfach und gleichmäßig ins Haar einmassieren und "bricht" hervorragend am Haar. Dadurch kann er sich besonders vorteilhaft um den Haarschaft legen, was unter anderem zu einer Verbesserung der Kämmbarkeit und des Griffes von Haaren im nassen und trockenen Zustand führt. Auf diese Weise lassen sich Haarfestiger mit guten Schaum- und Pflegeeigenschaften zubereiten.

Erfindungsgemäß ist die Konzentration eines Terpolymers aus Vinylpyrrolidon, Dimethylaminopropylmethacrylamid und quaternisiertem Alkyldimethylaminopropylmethacrylamid von 0,1 bis 10 Gewichts-% und insbesondere erfindungsgemäß vorteilhaft von 0,25 bis 2,5 Gewichts-%, jeweils bezogen auf das Gesamtgewicht der Zubereitung.

Erfindungsgemäß besonders vorteilhaft ist die Konzentration mindestens einer kationischen Substanz von 0,05 bis 5 Gewichts-% und insbesondere von 0,1 bis 1 Gewichts-%, jeweils bezogen auf das Gesamtgewicht der Zubereitung.

Die erfindungsgemäß vorteilhaften Terpolymere bestehen aus Vinylpyrrolidon, Dimethylamino-propylmethacrylamid und Alkyldimethylaminopropylmethacrylamidoammoniumsalzen.

Alle in der WO 00 68282 offenbarten Terpolymere sind erfindungsgemäß vorteilhaft.

Ganz besonders vorteilhaft ist dabei das Terpolymer aus Vinylpyrrolidon, Dimethylaminopropylmethacrylamid und Lauryldimethylaminopropylmethacrylamidoammoniumchlorid (z.B. Styleze W-20 der Firma ISP).

Die erfindungsgemäßen Haarfestiger werden erfindungsgemäß mit einem Treibgas aufgeschäumt. Dieses wird erfindungsgemäß in einer Menge von 0,5 bis 20 Gewichts-%, besonders vorteilhaft in einer Menge von 5 bis 15 und ganz besonders vorteilhaft in einer Menge von 8 bis 11 Gewichts-%, jeweils bezogen auf das Gesamtgewicht der Formulierung eingesetzt. Erfindungsgemäße Treibgase sind Propan, Isobutan und n-Butan sowie deren Mischungen.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Die kosmetischen und dermatologischen Zubereitungen können erfindungsgemäß kosmetische Hilfsstoffe enthalten, wie sie üblicherweise in solchen Zubereitungen verwendet werden, z.B. Konservierungsmittel, Antioxidanien, Bakterizide, Parfüme, Emulgatoren, weichmachende, anfeuchtende und/oder feuchthaltende Substanzen, Fette, Öle, Wachse, Farbstoffe, Pigmente, die eine färbende Wirkung haben, Verdickungsmittel oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate. Auch die Einarbeitung von UV-Lichtschutzfiltern ist erfindungsgemäß vorteilhaft. Die aufgeführte Liste an Zusatzstoffen soll selbstverständlich nicht limitierend sein.

Eine besonders bevorzugte Ausführungsform der erfindungsgemäßen Haarfestiger ist ihr Vorliegen in Form einer wässrig und/oder alkoholischen Lösung.

Die erfindungsgemäßen Haarfestiger werden erfindungsgemäß vorteilhaft in einer Aerosolverpackung als Schaumspender verpackt, aus der heraus sie vorteilhaft angewendet werden können.

Auch sind Schaumspender auf Aerosolverpackungsbasis, welche einen erfindungsgemäßen Haarfestiger enthalten, erfindungsgemäß.

Erfindungsgemäß ist weiterhin die Verwendung erfindungsgemäßen Haarfestigers als Schaumfestiger.

Außerdem ist die Verwendung kationischer Substanzen zur Verbesserung der Schaumeigenschaften kosmetischen und/oder dermatologischer Zubereitungen enthaltend Terpolymer aus Vinylpyrrolidon, Dimethylaminopropylmethacrylamid und quaternisiertem Alkyldimethylaminopropylmethacrylamid sowie die Verwendung von Terpolymer aus Vinylpyrrolidon, Dimethylaminopropylmethacrylamid und quaternisiertem Alkyldimethylaminopropylmethacrylamid zur Verbesserung der Schaumeigenschaften kosmetischer und/oder dermatologischer Zubereitungen enthaltend kationische Substanzen erfindungsgemäß.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| | **1** | **2** | **3** |
|---|---|---|---|
| Vinylpyrrolidon, Dimethylamino-propylmethacrylamid, Lauryldimethylamino-propylmethacrylamidoammoniumchlorid, (Styleze W-20 der Firma ISP) | 2 | 1 | 0,75 |
| Cetyltrimethylammoniumchlorid | 0,3 | 0,4 | 0,5 |
| Parfüm, Lösungsvermittler, pH-Einstellung, Konservierung | q.s. | q.s. | q.s. |
| Propan/Butan | 10 | 10 | 10 |
| Wasser | ad 100 | ad 100 | ad 100 |

## Patentansprüche

1. Haarfestiger enthaltend
a) mindestens ein Terpolymer aus Vinylpyrrolidon, Dimethylaminopropylmethacrylamid und quaternisiertem Alkyldimethylaminopropylmethacrylamid in einer Konzentration von 0,1 bis 10 Gewichts-%
b) mindestens eine kationische Substanz in einer Konzentration von 0,01 bis 20 Gewichts-%,
jeweils bezogen auf das Gesamtgewicht der Zubereitung, **dadurch gekennzeichnet, dass** die kationische Substanz ein kationisches, betainisches oder amphoteres Tensid, eine Silikonverbindung mit kationischen Gruppen, Cetyltrimethylammoniumchlorid und/oder Cetyltrimethylammoniumphosphat, ein kationisch derivatisiertes Protein oder ein kationisch derivatisiertes hydrolysiertes Protein ist sowie **dadurch gekennzeichnet, dass** die Zubereitung mit einem Treibgas aufgeschäumt wird, wobei als Treibgas Propan, Isobutan und/oder n-Butan eingesetzt wird.

2. Haarfestiger nach einem der Ansprüche 1, **dadurch gekennzeichnet, dass** das Treibgas in einer Menge von 0,5 bis 10 Gewichts-% bezogen auf das Gesamtgewicht der Formulierung eingesetzt wird.

3. Haarfestiger nach einem der Ansprüche 1 bis 2, **dadurch gekennzeichnet, dass** die Zubereitung weitere Wirk-, Hilfs und/oder Zusatzstoffe enthält.

4. Haarfestiger nach einem der Ansprüche 1 bis 3, **dadurch gekennzeichnet, dass** die Zubereitung in einer Aerosolverpackung als Schaumspender vorliegt, aus der heraus sie angewendet wird.

5. Schaumspender auf Aerosolverpackungsbasis, enthaltend einen Haarfestiger nach einem der Ansprüche 1 bis 4.

6. Verwendung eines Haarfestigers nach einem der vorhergehenden Ansprüche als Schaumfestiger.

## Claims

1. Hair fixative composition comprising
a) at least one terpolymer of vinylpyrrolidone, dimethylaminopropylmethacrylamide and quaternized alkyldimethylaminopropylmethacrylamide in a concentration of from 0.1 to 10% by weight
b) at least one cationic substance in a concentration of from 0.01 to 20% by weight,
in each case based on the total weight of the preparation, **characterized in that** the cationic substance is a cationic, betainic or amphoteric surfactant, a silicone compound with cationic groups, cetyltrimethylammonium chloride and/or cetyltrimethylammonium phosphate, a cationically derivatized protein or a cationically derivatized hydrolysed protein, and **characterized in that** the preparation is foamed with a propellant gas, where the propellant gas used is propane, isobutane and/or n-butane.

2. Hair fixative composition according to Claim 1, **characterized in that** the propellant gas is used in an amount of from 0.5 to 10% by weight, based on the total weight of the formulation.

3. Hair fixative composition according to one of Claims 1 to 2, **characterized in that** the preparation comprises further active ingredients, auxiliaries and/or additives.

4. Hair fixative composition according to one of Claims 1 to 3, **characterized in that** the preparation is in the form of an aerosol packaging as foam dispenser from which it is applied.

5. Foam dispenser on an aerosol packaging basis comprising a hair fixative composition according to one of Claims 1 to 4.

6. Use of a hair fixative composition according to one of the preceding claims as foam fixative composition.

## Revendications

1. Fixateur pour cheveux, contenant
a) au moins un terpolymère de vinylpyrrolidone, diméthylaminopropylméthacrylamide et alkyldiméthylaminopropylméthacrylamide rendu quaternaire, à une concentration de 0,1 à 10 % en poids
b) au moins une substance cationique à une concentration de 0,01 à 20 % en poids,
chaque fois par rapport au poids total de la préparation, **caractérisé en ce que** la substance cationique est un tensioactif cationique, de type bétaïne ou amphotère, un composé silicone à groupes cationiques, le chlorure de cétyltriméthylammonium et/ou le phosphate de cétyltriméthylammonium, une protéine à fonctionnalisation cationique ou une protéine hydrolysée à fonctionnalisation cationique, et **caractérisé en ce que** la préparation est transformée en mousse à l'aide d'un gaz propulseur, le propane, l'isobutane et/ou le n-butane étant utilisé(s) en tant que gaz propulseur.

2. Fixateur pour cheveux selon la revendication 1, **caractérisé en ce que** le gaz propulseur est utilisé en une quantité de 0,5 à 10 % en poids, par rapport au poids total de la composition.

3. Fixateur pour cheveux selon la revendication 1 ou 2, **caractérisé en ce que** la préparation contient d'autres actifs, adjuvants et/ou additifs.

4. Fixateur pour cheveux selon l'une quelconque des revendications 1 à 3, **caractérisé en ce que** la préparation se trouve dans un emballage aérosol sous forme de distributeur de mousse, à partir duquel elle est appliquée.

5. Distributeur de mousse à base d'un emballage aérosol, contenant un fixateur pour cheveux selon l'une quelconque des revendications 1 à 4.

6. Utilisation d'un fixateur pour cheveux selon l'une quelconque des revendications précédentes, en tant que fixateur en mousse.
